# EUROPÄISCHE PATENTSCHRIFT

(11) **EP 2 014 250 B1**
(45) Veröffentlichungstag und Bekanntmachung des Hinweises auf die Patenterteilung: **15.09.2010**
(21) Anmeldenummer: 08011892.0
(22) Anmeldetag: 02.07.2008
(51) Int. Cl.: A61B 18/14, A61B 18/00

(54) **Medizinisches Instrument zum Koagulieren von Gewebe**
Medical instrument for coagulating tissue
Instrument médical destiné à coaguler des tissus

(30) Priorität: 13.07.2007 DE 102007034578
(43) Veröffentlichungstag der Anmeldung: 14.01.2009
(73) Patentinhaber: Karl Storz GmbH & Co. KG, 78532 Tuttlingen (DE)
(72) Erfinder: Eitenmüller, Jürgen P., Prof. Dr. med., 44575 Castrop-Rauxel (DE)
(74) Vertreter: Heuckeroth, Volker

(56) Entgegenhaltungen:
- US-A- 5 403 311
- US-A- 5 451 223
- US-A- 6 032 673

## Beschreibung

Die Erfindung betrifft ein medizinisches Instrument zum Koagulieren von Gewebe, mit einem Elektrodenträger und mit einer mit Hochfrequenzstrom beaufschlagbaren Elektrode an einem distalen Ende des Elektrodenträgers, wobei die Elektrode rohrförmig ausgebildet ist, und mit einem Abstreifer, der unmittelbar benachbart zur Elektrode angeordnet ist, wobei der Abstreifer und die Elektrode zum Entfernen von Verschmutzungen an der Elektrode relativ zueinander bewegbar sind.

Ein solches medizinisches Instrument ist aus dem Dokument DE 33 90 567 C2 bekannt.

Solche medizinischen Instrumente werden bspw. im Bereich der minimal-invasiven Chirurgie während eines laparoskopischen Eingriffs zum Veröden von Gewebe verwendet. Das medizinische Instrument wird dabei durch einen Trokar hindurch in eine Körperöffnung eines Patienten eingeführt, so dass die mit Hochfrequenzstrom beaufschlagbare Elektrode am distalen Ende des Elektrodenträgers mit dem zu koagulierenden Gewebe in Berührung kommt und dieses verödet.

Aus dem Firmenprospekt der Karl Storz GmbH & Co., Tuttlingen, "Karl Storz-Endoskope, Laparoskopie", 5. Ausgabe 1/2005, Seite 182, ist ein mit der Produktnummer 37370 SC versehenes medizinisches Instrument für die Laparoskopie bekannt.

Das bekannte medizinische Instrument weist einen Elektrodenträger in Form eines Koagulations-Saugrohrs auf, an dessen distalem Ende eine rohrförmige Elektrode aus Metall angeordnet ist. Der Elektrodenträger ist an seinem proximalen Ende über einen an einer Kupplung angeordneten Anschluss mit Hochfrequenzstrom beaufschlagbar. Während des Eingriffs kann ferner eine Saugleitung an das proximale Ende des als Saugrohr ausgebildeten Elektrodenträgers angeschlossen werden, so dass bspw. Blut oder Flüssigkeit aus dem Operationsgebiet nach proximal abgesaugt wird.

Nachteilig an diesem medizinischen Instrument ist es, dass sich während der Gewebekoagulation Verschmutzungen in Form von bspw. verbranntem Gewebe an der als Koagulationsfläche wirkenden Außenfläche der Elektrode ablegen, wodurch die Elektrode im Laufe der Zeit mit diesen oftmals hartnäckig anhaftenden Ablagerungen belegt ist. Diese an der Elektrode anhaftenden Verschmutzungen bilden eine isolierende Schicht auf der metallenen Elektrodenoberfläche, so dass die wirksame Koagulationsfläche der Elektrode verkleinert bzw. vollständig eliminiert wird und nicht mehr mit dem zu verödenden Gewebe in Berührung kommt. Hierdurch ist das medizinische Instrument für einen weiteren Gebrauch ungeeignet.

Es ist ferner nachteilig, dass die mit Verschmutzungen belegte Elektrode des medizinischen Instruments nur außerhalb des Patienten gereinigt werden kann, so dass das medizinische Instrument aus der Körperöffnung des Patienten entnommen und nach einem Reinigen bzw. Austauschen der Elektrode erneut in die Körperöffnung des Patienten eingeführt werden muss. Hierdurch gestaltet sich der medizinische Eingriff zeitaufwändig.

Das aus dem eingangs genannten Dokument DE 33 90 567 C2 bekannte medizinische Instrument ist ein Bipolar-Elektrokoagulator, der am distalen Ende eine rohrförmig ausgebildete Elektrode aufweist. Die Elektrode weist auf ihrer Außenfläche eine schraubenförmige Nut auf. Am Außenumfang der Elektrode ist ein Abstreifer angeordnet, der dazu dient, Koagulat von den erhabenen Bereichen der Elektrode abzustreifen und in die schraubenförmige Nut zu verdrängen, über die das Koagulat nach proximal transportiert wird. Die Elektrode ist an ihrem distalen Ende geschlossen.

Aus DE 696 35 288 T2 sind elektrochirurgische Elektroden bekannt, die eine Silikonbeschichtung aufweisen, die den Aufbau von Koagulat verhindert und das Entfernen desselben erleichtert.

Aus US 5,460,629 ist ein elektrochirurgisches Instrument bekannt, das am distalen Ende zwei Arten von Elektroden aufweist, und zwar eine hakenförmige Elektrode und zwei seitlich zur hakenförmigen Elektrode angeordnete plattenförmige Elektroden. Die plattenförmigen Elektroden können axial relativ zur hakenförmigen Elektrode verschoben werden, wobei durch das Vorschieben der plattenförmigen Elektroden anhaftendes verkohltes Gewebe von der hakenförmigen Elektrode abgestreift werden kann. Die plattenförmigen Elektroden wirken jedoch nur an den Seitenflächen der hakenförmigen Elektrode.

US 5,451,223 offenbart ein elektrochirurgisches Skalpell mit einer Klinge, die eine Klingenreinigungsvorrichtung aufweist. Die Reinigungsvorrichtung wird durch eine Metallhülse gebildet, deren Innenquerschnitt an den Außenquerschnitt der Klinge angepasst ist, und deren distale Mündung als scharfe Klinge ausgebildet ist. Durch axiales Vorschieben der Hülse wird an der Klinge anhaftendes Koagulat abgestreift.

Aus US 6,032,673 ist ein elektrochirurgisches Schneidinstrument bekannt, bei dem am distalen Ende ein Schneidkopf angeordnet ist, der um die Längsachse des Schafts drehbar ist. Der Schneidkopf weist mehrere Schneiden auf, die in Umfangsrichtung um die Drehachse des Schneidkopfes orientiert sind. Der Schneidkopf ist in einem seitlich offenen distalen Ende des Schafts angeordnet. Die mehreren Schneiden weisen jeweils eine Öffnung auf, deren Schneidkante mit dem Rand der Öffnung des distalen Endes des Schafts schneidend zusammenwirkt.

In US 5,403,311 ist ein elektrochirurgisches Koagulationsinstrument in Form eines Katheters offenbart, der einen Katheterkörper und eine erste Elektrode aufweist, die in Form eines Tellers ausgebildet ist, und die eine Öffnung aufweist, durch die eine Sonde nach distal vorsteht, die eine hohle Nadel enthält, die die Gegenelektrode bildet. Um zu vermeiden, dass sich an der Sonde koaguliertes Blut und Gewebe anlagern kann, kann eine Isolierhülse zurückgezogen werden, um die freiliegende Oberfläche der leitenden Sonde zu erhöhen und damit die Stromdichte zu verringern. In einem anderen Beispiel wird an der Nadel anhaftendes koaguliertes Gewebe oder Blut durch Zurückziehen der Nadel zusammen mit der vorstehend genannten Isolierhülse in den Katheterkörper an der Öffnung der Elektrode abgestreift. Bei dieser Anordnung bildet somit die mittige Öffnung der Elektrode einen Abstreifer für die Nadel.

Schließlich offenbart EP 0 418 382 A1 ein elektrochirurgisches Instrument mit Elektroden, denen Reinigungselemente zugeordnet sind, die relativ zu den Elektroden axial beweglich, bogenförmig beweglich, kreisförmig beweglich, usw. sein können. Zum Bewegen der Reinigungselemente ist ein elektrischer Antrieb vorgesehen.

Die zuvor beschriebenen bekannten elektrochirurgischen medizinischen Instrumente weisen allesamt am distalen Ende geschlossene Elektroden auf. Zum Spülen oder Absaugen werden zusätzliche Saug- und/oder Spülrohre benötigt.

Es ist eine Aufgabe der vorliegenden Erfindung, ein medizinisches Instrument der eingangs genannten Art derart zu verbessern, dass es zum Spülen und/oder Absaugen geeignet ist und die Elektrode während des gesamten medizinischen Eingriffs ihre Koagulationswirkung aufrecht erhält.

Erfindungsgemäß wird die Aufgabe hinsichtlich des eingangs genannten medizinischen Instruments dadurch gelöst, dass die Elektrode an einem distalen Ende eine Öffnung aufweist, und dass der Abstreifer das distale Ende der Elektrode umgreift.

Das medizinische Instrument weist an dem distalen Ende seines Elektrodenträgers einen Abstreifer zum Entfernen von an der Elektrode anhaftenden Verschmutzungen auf, der sich in unmittelbarer Nähe zur Elektrode befindet. Erfindungsgemäß ist unter einer "unmittelbar benachbarten Anordnung" der Elektrode und des Abstreifers zu verstehen, dass sich der Abstreifer und die Elektrode berühren bzw. in einem derart geeigneten Abstand zueinander angeordnet sind, dass die Elektrode und der Abstreifer zum Entfernen der an der Elektrode anhaftenden Verschmutzungen zusammenwirken können. Die an der Elektrode anhaftenden Verschmutzungen werden dadurch entfernt, dass der Abstreifer und die Elektrode relativ zueinander bewegt werden und der Abstreifer die Verschmutzungen an der Elektrode abstreift. Die Relativbewegung des Abstreifers und der Elektrode zueinander kann axial oder quer zur axialen Richtung erfolgen. Auf diese Weise wird vorteilhafterweise gewährleistet, dass die Elektrode des medizinischen Instruments während des medizinischen Eingriffs gereinigt werden kann, wodurch die sich während des Eingriffs auf der Elektrode bildende isolierende Verschmutzungsschicht entfernt wird.

Dadurch muss der Gewebekoagulationsvorgang nicht unterbrochen werden, um die Elektrode von den an ihrer Koagulationsfläche anhaftenden Verschmutzungen zu befreien. Vielmehr können das Veröden des Gewebes und das Reinigen der Elektrode gleichzeitig durchgeführt werden, da der Abstreifer und die Elektrode unmittelbar zueinander benachbart angeordnet sind und nicht bspw. der Abstreifer zur Elektrode hin verschoben werden muss, um diese zu reinigen.

Der medizinische Eingriff gestaltet sich besonders zeitsparend, da keine Entnahme der mit Verschmutzungen belegten Elektrode aus der Körperöffnung des Patienten erforderlich ist. Vielmehr ermöglicht die reinigbare Elektrode am distalen Ende des medizinischen Instruments ein kontinuierliches Arbeiten des Operateurs ohne Unterbrechungen.

Dadurch, dass die Elektrode rohrförmig mit einer Öffnung am distalen Ende ausgebildet ist, kann das medizinische Instrument nicht nur zum Koagulieren, sondern auch als Saug- und Spülinstrument verwendet werden, wodurch beispielsweise Blut oder abgeschabtes Gewebe durch das Saugrohr aus dem Operationsgebiet abgesaugt bzw. das Operationsgebiet durch Spülflüssigkeit, die entlang des Elektrodenträgers von seinem proximalen Ende zu seinem distalen Ende transportiert wird, gesäubert werden kann.

Dadurch, dass der Abstreifer das distale Ende der Elektrode umgreift, d.h., dass der Abstreifer von der Außenseite der Elektrode kommend in die Öffnung der Elektrode eingreift, wird auch am stirnseitigen Rand der Öffnung der Elektrode anhaftendes Koagulat wirksam entfernt. Darüber hinaus besteht der Vorteil, dass der Abstreifer stets lagestabil an der Elektrode angeordnet ist, da das Umgreifen des distalen Endes der Elektrode durch den Abstreifer beispielsweise das Abheben und/oder Abbrechen des Abstreifers von der Elektrode durch nicht glatt, sondern klumpenartig an der Elektrodenoberfläche anhaftende Verschmutzungen verhindert wird. Ferner ermöglicht diese Ausgestaltung des Abstreifers vorteilhafterweise die Bereitstellung eines ausreichend großen Anlagedruckes des Abstreifers an der Elektrodenfläche entlang seiner gesamten Ausdehnung, so dass auch hartnäckig an der Elektrode anhaftende Verschmutzungen durch den Abstreifer entfernt werden können. Insbesondere wird hierdurch auch der distale stirnseitige Öffnungsrand der Elektrode wirksam vom Koagulat befreit. Die Elektrode erhält somit ihre Koagulationswirkung insbesondere auch an der Elektrodenspitze aufrecht.

In einer bevorzugten Ausgestaltung der Erfindung sind der Abstreifer und die Elektrode quer zu einer Längsrichtung des Elektrodenträgers relativ zueinander bewegbar.

Diese Maßnahme hat den Vorteil, dass das Koagulieren während der Reinigung der Elektrode nicht unterbrochen werden muss, da sich der Abstreifer und die Elektrode quer zur Längsrichtung des Elektrodenträgers und damit quer zur Elektrode bewegen und die Elektrode nicht zeitweise vollständig durch den Abstreifer verdeckt wird und stets eine gleichbleibend große Elektrodenfläche zur Koagulation vorhanden ist. Ferner ist die Bauweise des medizinischen Instruments vorteilhafterweise besonders stabil, da die fehlende axiale Verschiebbarkeit der Elektrode und des Abstreifers relativ zueinander bspw. ein axiales Abheben des distalen Endes des Abstreifers von der Elektrode aufgrund von klumpenartig erhöhten Verschmutzungen an der Elektrodenfläche und somit ein eventuelles Abbrechen des Abstreifers verhindert.

In einer weiteren bevorzugten Ausgestaltung der Erfindung erstreckt sich der Abstreifer in Längsrichtung zumindest entlang eines Teilbereichs der Elektrode.

Diese Maßnahme bewirkt, dass der Abstreifer und seine der Elektrode zugewandte wirksame Reinigungsfläche ausreichend groß dimensioniert sind, wodurch eine großflächige Reinigung der Elektrode ermöglicht wird. Hierdurch wird vorteilhafterweise das Reinigen der Elektrode durch den Abstreifer besonders schnell durchgeführt. Vorzugsweise erstreckt sich der Abstreifer entlang der gesamten Elektrodenlänge.

In einer weiteren bevorzugten Ausgestaltung der Erfindung verjüngt sich der Abstreifer nach distal hin.

Diese Maßnahme hat den Vorteil, dass die wirksame Koagulationsfläche der Elektrode nicht unnötig durch den Abstreifer verkleinert wird, so dass die Koagulationsfläche der Elektrode besonders groß ist und der medizinische Eingriff zügig durchgeführt werden kann.

In einer weiteren bevorzugten Ausgestaltung der Erfindung weist der Abstreifer eine der Elektrode zugewandte Schabkante auf, die als Messer ausgebildet ist.

Diese Maßnahme bewirkt, dass die die Elektrode reinigende Wirkung des Abstreifers durch ein Schaben der Schabkante an der Elektrodenoberfläche erreicht wird, wodurch das benachbart zur Schabkante an der Elektrode anhaftende Gewebe von der Elektrodenoberfläche abgekratzt und somit entfernt wird. Hierdurch wird eine besonders gründliche Reinigung der Elektrode durch den Abstreifer erreicht. Die Ausgestaltung der Schabkante als Messer ermöglicht vorteilhafterweise ein gezieltes Abtragen der Verschmutzungen in einem definierten Bereich entlang der Schneidkante des Messers.

In einer weiteren bevorzugten Ausgestaltung der Erfindung weist der Abstreifer zwei der Elektrode zugewandte Schabkanten auf, die jeweils als Messer ausgebildet sind.

Diese Maßnahme hat den Vorteil, dass die an der Elektrode angelagerten Verschmutzungen je nach Anordnung der Schabkanten bspw. gleichzeitig an zwei Bereichen, die jeweils benachbart zu einer der Schabkante liegen, oder auch in zwei verschiedenen Bewegungsrichtungen von Abstreifer und Elektrode von der Elektrodenoberfläche abgeschabt werden können, so dass die Reinigung der Elektrode beschleunigt wird.

In einer weiteren bevorzugten Ausgestaltung ist der Abstreifer plattenförmig, wobei der Abstreifer die Elektrode teilumfänglich umgibt.

Diese Maßnahme bewirkt, dass durch die rohrförmige Elektrode eine größtmögliche und vollumfängliche Koagulationsfläche bereitgestellt wird und gleichzeitig der plattenförmige Abstreifer derart klein bemessen ist, dass er die Koagulationsfläche der Elektrode nicht wesentlich reduziert. Somit bleibt vorteilhafterweise die koagulierende Wirkung des medizinischen Instruments fast vollständig erhalten und wird nicht wesentlich durch den Abstreifer eingeschränkt. Ferner stellt der plattenförmige Abstreifer entlang seinen in Längsrichtung des Elektrodenträgers verlaufenden Plattenkanten zwei Schabkanten bereit, so dass das Reinigen von den jeweils an den Schabkanten angrenzenden Elektrodenflächen in jeder der beiden Relativbewegungsrichtungen der Elektrode und des Abstreifers möglich ist.

Im Zusammenhang mit der die Elektrode umgreifenden Ausgestaltung des distalen Endes des Abstreifers sind die rohrförmige bzw. plattenförmige Gestalt der Elektrode und des Abstreifens besonders vorteilhaft, da das distale Ende des Abstreifers an dem Rand der Rohröffnung des distalen Endes der Elektrode eingehängt werden kann und der Abstreifer stets lagestabil an der Elektrode anliegt.

In einer weiteren bevorzugten Ausgestaltung der Erfindung umgibt der Abstreifer die rohrförmige Elektrode über einen Winkelbereich von weniger als etwa 60°, vorzugsweise über einen Winkelbereich von weniger als etwa 45° und weiter vorzugsweise über einen Winkelbereich von weniger als etwa 30°.

Diese Maßnahme bewirkt, dass einerseits der Abstreifer ausreichend groß bemessen ist, um ein schnelles Reinigen der Elektrodenoberfläche zu gewährleisten, und andererseits jedoch klein genug ist, um die Koagulationsfläche der Elektrode nicht unnötig zu reduzieren. Daher kann der medizinische Eingriff vorteilhafterweise zügig durchgeführt werden.

In einer weiteren bevorzugten Ausgestaltung der Erfindung weist das medizinische Instrument ein Außenrohr auf, an dessen distalem Ende der Abstreifer angeordnet ist, wobei der Elektrodenträger in dem Außenrohr aufgenommen ist, und wobei der Elektrodenträger und das Außenrohr relativ zueinander drehbar sind.

Diese Maßnahme ermöglicht vorteilhafterweise eine besonders kompakte und einfache Bauweise des medizinischen Instruments. Das Außenrohr umgibt den Elektrodenträger und schützt diesen vor Beschädigungen. Die relative Beweglichkeit der Elektrode und des Abstreifers wird durch die Relativdrehbarkeit des Elektrodenträgers und des Außenrohrs zueinander bewerkstelligt, wobei das Drehen von Elektrodenträger und Außenrohr relativ zueinander das Reinigen der gesamten Elektrodenfläche in kurzer Zeit ermöglicht.

In einer weiteren bevorzugten Ausgestaltung der Erfindung ist das Außenrohr drehfest und der Elektrodenträger relativ zum Außenrohr drehbar.

Der Elektrodenträger im Inneren des Außenrohrs wird zur Erzeugung der Relativdrehbewegung der Elektrode und des Abstreifers gedreht, wobei insbesondere das Drehen des Elektrodenträgers um seine Längsachse in beide Drehrichtungen möglich ist. Diese Maßnahme gewährleistet vorteilhafterweise, dass das Außenrohr relativ zum Trokar, durch den das medizinische Instrument üblicherweise während des Eingriffs in den Körper eingeführt wird, drehfest in seiner Position verbleibt, so dass zum einen die Reinigung der Elektrode mit dem Abstreifer nicht einer Reibung zwischen Außenrohr und Trokar unterliegt und außerdem die Dichtigkeit des Trokars durch eine Drehung des Außenrohrs nicht beeinträchtigt wird. Das Drehen des Elektrodenträgers und somit der Elektrode in die zwei Drehrichtungen resultiert vorteilhafterweise in einem schnellen und flexiblen Reinigen der Elektrodenoberfläche, da das an der Elektrodenoberfläche anhaftende Gewebe sofort durch entsprechende Drehung der Elektrode durch die in Drehrichtung vordere Schabkante entfernt werden kann. Eine Drehung der Elektrode um einen Winkel von 180° in beide Drehrichtungen genügt, die gesamte Elektrodenfläche zu reinigen, da die von der jeweiligen Schabkante aus gerechnete halbumfängliche Elektrodenfläche durch die in Drehrichtung vordere Schabkante gereinigt wird. Daher ist vorteilhafterweise keine vollumfängliche Drehbarkeit der Elektrode um 360° nötig, was in einer einfacheren Konstruktion des Drehmechanismuses des medizinischen Instruments resultiert.

In einer weiteren bevorzugten Ausgestaltung der Erfindung ist der Elektrodenträger als Saugrohr ausgebildet.

Diese Maßnahme hat den Vorteil, dass über die distale Öffnung der Elektrode abgesaugte Flüssigkeit und/oder Gewebe über den Elektrodenträger nach proximal befördert werden kann, ohne dass mit der Elektrode ein separates Saugrohr verbunden werden muss.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert.
Es zeigen:
Fig. 1 ein erfindungsgemäßes medizinisches Instrument;
Fig. 2 eine perspektivische Ansicht eines distalen Endes des medizinischen Instruments in Fig. 1;
Fig. 3 eine Querschnittszeichnung des distalen Endes des medizinischen Instruments in Fig. 1; und
Fig. 4 eine weitere perspektivische Ansicht des distalen Endes des medizinischen Instruments in Fig. 1.

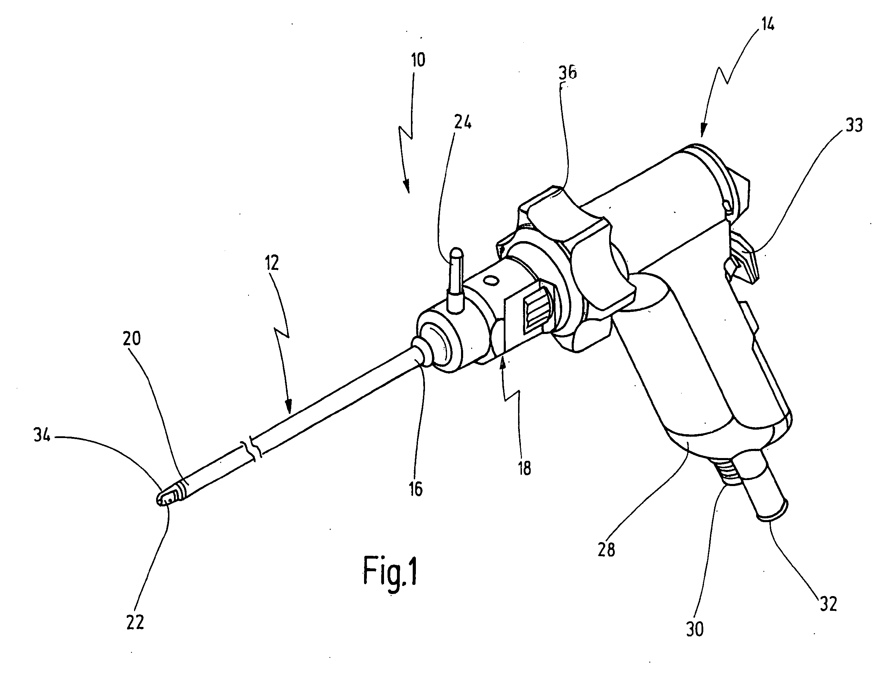

In Fig. 1 ist ein mit dem allgemeinen Bezugszeichen 10 versehenes medizinisches Instrument dargestellt. Das medizinische Instrument 10 dient im Bereich der minimal-invasiven Chirurgie während eines laparoskopischen Eingriffs zum Koagulieren von Gewebe in einem Operationsgebiet, zum Absaugen von bspw. Gewebeflüssigkeit oder Blut aus dem Operationsgebiet sowie zum Spülen des Operationsgebiets.

Das medizinische Instrument 10 weist einen starren Schaft 12 und einen Handgriff 14 auf. Ein proximales Ende 16 des Schafts 12 ist über eine Kupplung 18 mit dem Handgriff 14 verbindbar.

Der Schaft 12 weist an seinem distalen Ende 20 eine Elektrode 22 aus Metall auf. Die Elektrode 22 ist über einen im Bereich des proximalen Endes 16 des Schafts 12 angeordneten Anschluss 24 mit Hochfrequenzstrom beaufschlagbar. Dazu wird an dem Anschluss 24 eine Hochfrequenzspannungsquelle (nicht dargestellt) angeschlossen. Der Anschluss 24 ist an der am proximalen Ende 16 des Schafts 12 angeordneten Kupplung 18 zur Verbindung des Schafts 12 vorgesehen.

Der Handgriff 14 weist an seinem unteren Ende 28 einen Spülanschluss 30 und einen Sauganschluss 32 auf, die jeweils mit einem Saugschlauch (nicht dargestellt) bzw. einem Spülschlauch (nicht dargestellt) verbindbar sind. Der Handgriff 14 weist ferner eine Ventilbetätigung 33 zum Aktivieren und Regulieren des Saugstroms und der Spülflüssigkeitszufuhr durch den Saug- bzw. Spülanschluss 30, 32 auf.

Während des laparoskopischen Eingriffs kann sich koaguliertes Gewebe an der Elektrode 22 krustenartig mit hoher Haftkraft ablagern, das dann an der Elektrode 22 anhaftet, so dass sich eine isolierende Schicht an der Elektrode 22 bildet, die den weiteren Gebrauch des medizinischen Instruments 10 einschränkt oder gar unmöglich macht. Um diese isolierende Schicht zu entfernen, weist das medizinische Instrument 10 einen Abstreifer 34 auf, der benachbart zur Elektrode 22 am distalen Ende 20 des Schafts 12 angeordnet ist. Die Elektrode 22 und der Abstreifer 34 sind über ein am proximalen Ende 16 des Schafts 12 angeordnetes Drehrad 36 der Kupplung 18 relativ zueinander drehbar.

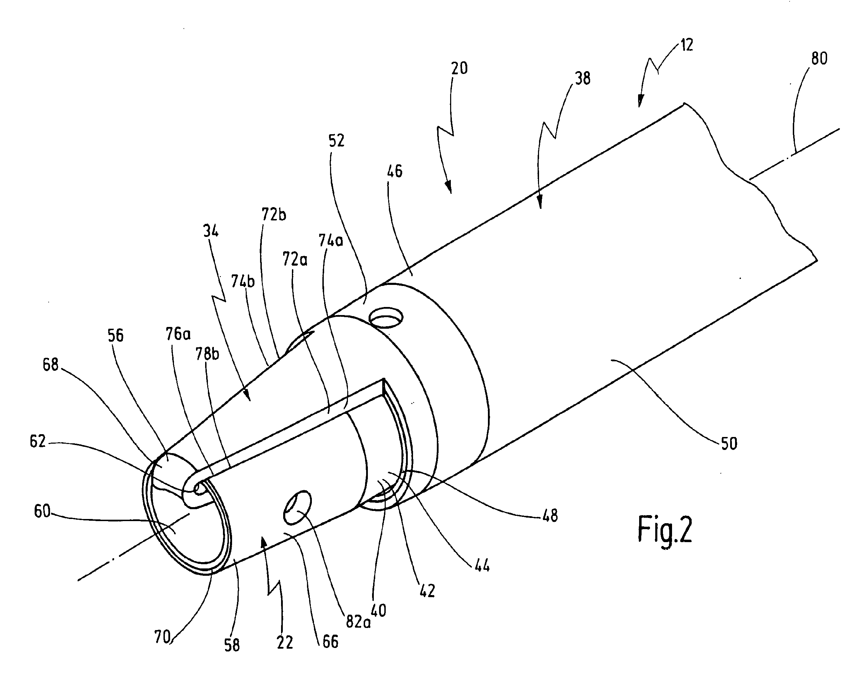

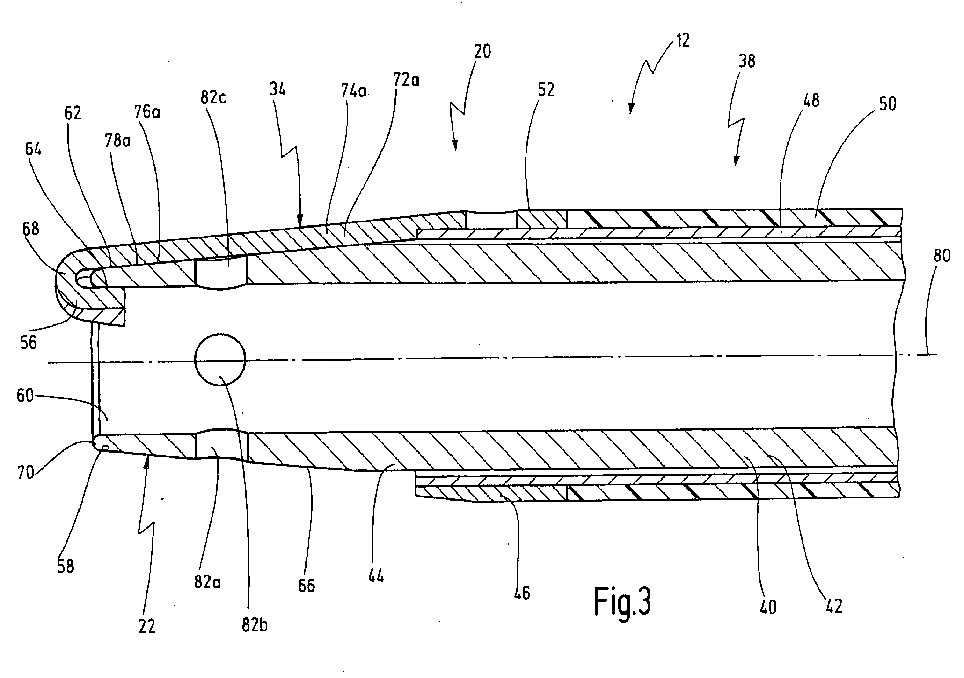

Fig. 2 und 3 zeigen eine perspektivische Ansicht bzw. eine Querschnittsansicht des distalen Endes 20 des medizinischen Instruments 10. Der Schaft 12 des medizinischen Instruments 10 weist ein Außenrohr 38 auf, das ein radial inneres Saugrohr 40 umgibt. Das Saugrohr 40 dient gleichzeitig als Elektrodenträger 42 für die Elektrode 22. Die Elektrode 22 ist an einem distalen Ende 44 des Saugrohrs 40 angeordnet, und der Abstreifer 34 ist an einem distalen Ende 46 des Außenrohrs 38 befestigt. Das Saugrohr 40 und die Elektrode 16 sind einstückig aus Metall gefertigt. Im Fall einer nicht-einstückigen Bauweise des Saugrohrs 40 und der Elektrode 22 können die beiden Bauteile auch aus verschiedenen Metallen gefertigt sein. Das Außenrohr 38 ist zweiteilig aufgebaut und weist ein vollumfänglich mit einer Kunststoffisolierung 50 ummanteltes inneres Rohr 48 auf. Das innere Rohr 48 des Außenrohrs 38 ist von dem Saugrohr 40 geringfügig beabstandet. Das Außenrohr 38 ist distalseitig kürzer als das Saugrohr 40 ausgebildet, so dass die am distalen Ende 44 des Saugrohrs 40 angeordnete Elektrode 22 aus dem Außenrohr 38 hervorragt. Der Abstreifer 34 ist über einen vollumfänglichen proximalen Ringbereich 52 an dem Außenrohr 38 befestigt.

Die Elektrode 22 ist konusförmig ausgebildet und verjüngt sich nach distal. Der Abstreifer 34 ist distalseitig seines proximalen Ringbereichs 52 plattenförmig ausgebildet und wölbt sich über einen Teil des Umfangs der Elektrode 22 über einen Winkelbereich von weniger als etwa 30°, bspw. um etwa 15° um die Elektrode 22. Vorzugsweise kann der Abstreifer 34 die Elektrode 22 über einen Winkelbereich von weniger als etwa 60° und weiter vorzugsweise über einen Winkelbereich von weniger als etwa 45° umgeben. Die Größe des Abstreifers 34 ist hierbei bspw. durch seine Formstabilität bei Biege- und Drehbeanspruchungen quer zu seiner Längserstreckung bestimmt. Diese Ausgestaltung des Abstreifers 34 ermöglicht vorteilhafterweise, dass die Koagulationsfläche der Elektrode 22 ausreichend groß ist und nicht durch den Abstreifer 34 unnötig reduziert wird.

Ein distaler Endbereich 56 des Abstreifers 34 umgreift hakenförmig ein distales Ende 58 der Elektrode 22, das als kreisförmige Öffnung 60 ausgebildet ist. Hierbei liegt eine Innenfläche 62 des Abstreifers 34 im distalen Endbereich 56 an der Innenfläche 64 des distalen Endes 58 der Elektrode 22 an. Die zur Elektrode 22 gewandte Innenfläche 62 des Abstreifers 34 berührt ferner eine Außenfläche 66 der Elektrode 22 entlang der gesamten Längserstreckung der Elektrode 22. Es ist ebenfalls möglich, dass sich der Abstreifer 34 und die Elektrode 22 nicht berühren, sondern in einem Abstand zueinander angeordnet sind, der noch geeignet ist, die Reinigungswirkung des Abstreifers 34 zu erhalten. Ein äußerstes distales Ende 68 des Abstreifers 34 ist im Bereich der um etwa 180° verlaufenden Umbiegung des distalen Endes 56 des Abstreifers 34 innenseitig von einem abgerundeten Öffnungsrand 70 der Öffnung 60 der Elektrode 22 beabstandet. Der Abstreifer 34 verjüngt sich ferner nach distal hin und weist die Gestalt eines gekrümmten Trapezes auf, so dass die Koagulationsfläche der Elektrode 22 im Bereich ihres sich verjüngenden distalen Endes 58 ebenfalls ausreichend groß bleibt.

Der Innenradius der Elektrode 22 und des Saugrohrs 40 sind gleich bemessen, so dass distalseitig eine maximale Saugleistung des Saugrohrs 40 erhalten bleibt und ausreichend Spülflüssigkeit durch ein Saugrohrinneres in das Operationsgebiet befördert werden kann. Der Außenradius der Elektrode 22 verkleinert sich aufgrund der sich verjüngenden Form der Elektrode 22 nach distal hin.

Längskanten 72a, b des Abstreifers 34 sind als Schabkanten 74a, b ausgebildet, um ein Entfernen von auf der Außenfläche 66 der Elektrode 22 angelagertem Gewebe zu ermöglichen. Die Schabkanten 74a, b sind an ihren zur Außenfläche 66 der Elektrode 22 weisenden Enden 76a, b scharf, hier bspw. als Messer 78a, b, ausgebildet. Hierbei ist es möglich, dass nur die als Messer 78a, b ausgebildeten Schabkanten 74a, b an der Außenfläche 66 der Elektrode 22 anliegen bzw. unmittelbar benachbart zu ihr angeordnet sind, während die Innenfläche 62 des Abstreifers 34 von der Außenfläche 66 der Elektrode 22 beabstandet ist. Es ist ebenfalls möglich, dass die Innenfläche 62 des Abstreifers 34 eine abrasive Oberfläche mit geeigneter Oberflächenrauhigkeit aufweist, die ein Abtragen von an der Außenfläche 66 der Elektrode 22 anhaftendem Gewebe ermöglicht.

Der Abstreifer 34 ist aus bspw. nicht-leitendem Metall gefertigt. Er kann auch aus jedem anderen Material, bspw. Kunststoff, gefertigt sein, das seine Formstabilität unter Biege- und Drehbeanspruchungen quer zu seiner Längserstreckung gewährleistet. Es ist auch möglich, dass der Abstreifer 34 aus Kunststoff und die Messer 78a, b aus Metall gefertigt sind.

Das Saugrohr 40 und das Außenrohr 38 des medizinischen Instruments 10 sind relativ zueinander drehbar ausgebildet, so dass die Abschabwirkung des Abstreifers 34 dadurch erzielt wird, dass der Abstreifer 34 an der Außenfläche 66 der Elektrode 22 angreift und das dort anhaftende Gewebe oder sonstige Verschmutzungen abträgt. In dem gezeigten Ausführungsbeispiel ist das Außenrohr 38 drehfest ausgebildet, während das Saugrohr 40 in zwei Drehrichtungen um eine Längsachse 80 des medizinischen Instruments 10 gedreht werden kann. Die Längsachse 80 des medizinischen Instruments 10 entspricht der Längsrichtung des starren Schafts 12 des medizinischen Instruments 10. Es ist ebenfalls möglich, dass das Saugrohr 40 drehfest ausgebildet ist und sich das Außenrohr 38 um die Längsachse 80 des medizinischen Instruments 10 dreht.

Eine Drehbewegung des Saugrohrs 40 wird durch das am proximalen Ende 16 des Schafts 12 angeordnete, manuell betätigbare Drehrad 36 bewirkt, an dem die Bedienungsperson angreift. Die beabstandete Anordnung des Saugrohrs 40 und des inneren Rohrs 48 des Außenrohrs 38 verhindert, dass die Drehbewegung des Saugrohrs 40 um die Längsachse 80 des medizinischen Instruments 10 durch zusätzliche Reibkräfte zwischen aneinander anliegenden Flächen des Saugrohrs 40 und des Außenrohrs 38 beeinträchtigt wird.

Die Elektrode 22 weist ferner in ihrer Außenfläche 66 vier Öffnungen 82, in Fig. 3 davon dargestellt: drei Öffnungen 82a-c, auf, die jeweils unter 90° zueinander angeordnet sind. Die Öffnungen 82 ermöglichen ein Absaugen von seitlich der Elektrode 22 befindlicher Flüssigkeit oder Gewebe nach proximal. Durch die Öffnungen 82 kann ebenfalls die Spülflüssigkeit ausströmen und das Operationsgebiet seitlich der Elektrode 22 spülen. Die austretende Spülflüssigkeit benetzt auch die Außenfläche 66 der Elektrode 22 und kann die Verschmutzungen auf der Außenfläche 66 der Elektrode 22 einweichen bzw. anlösen, so dass das Abschaben der Verschmutzungen durch den Abstreifer 34 unterstützt wird und leichter erfolgen kann. Bei einer Drehung der Elektrode 22 um die Längsachse 80 des medizinischen Instruments 10 kommt die Spülflüssigkeit mit der benachbart zu der jeweiligen Öffnung 82 und entgegen der Drehrichtung des Saugrohrs 40 liegenden Außenfläche 66 der Elektrode 22 in Kontakt, so dass die dort anhaftenden Verschmutzungen angelöst werden, bevor sich diese Fläche mit dem angelösten Gewebe zu der jeweiligen in der Drehrichtung vorderen Schabkante 74a, b hindreht und das Gewebe durch das Messer 78a, b abgekratzt wird. Tritt die Spülflüssigkeit insbesondere durch eine der Öffnungen 82 aus, die sich im Bereich des Abstreifers 34 befindet, so vermindert die austretende Spülflüssigkeit die zwischen der Innenfläche 62 des Abstreifers 34 und der Außenfläche 66 der Elektrode 22 auftretende Reibung, wodurch ein Materialverschleiß der Elektrode 22 und des Abstreifers 34 verringert wird.

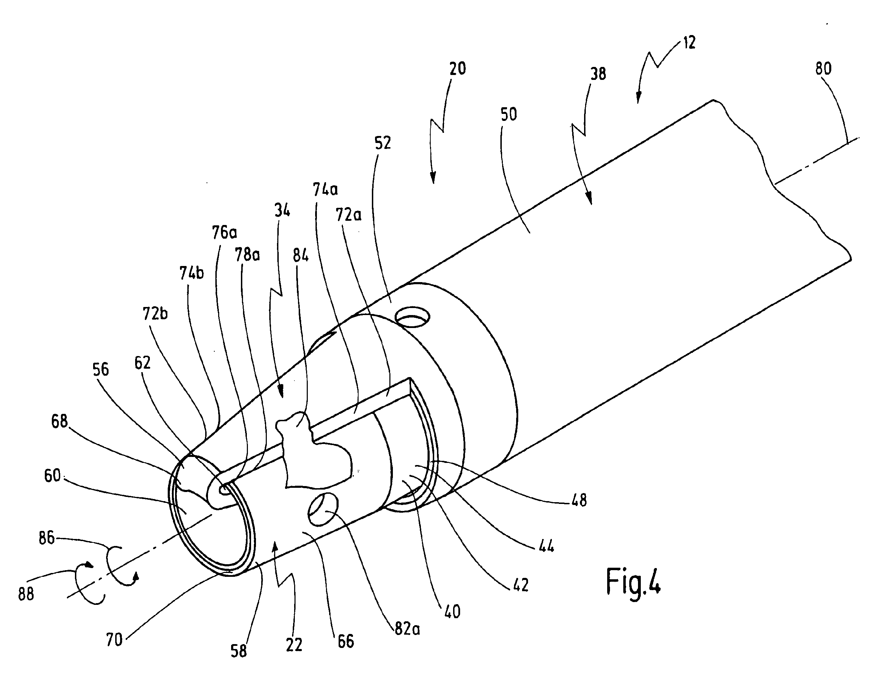

Fig. 4 zeigt das distale Ende 20 des Schafts 12 des medizinischen Instruments 10 während eines Betriebs. Die mit dem Bezugszeichen 84 dargestellte Verschmutzung in Form von auf der Außenfläche 66 der Elektrode 22 anhaftender Kruste wird durch das Drehen des Saugrohrs 40 um die Längsachse 80 des medizinischen Instruments 10 entlang eines Pfeils 86 entfernt. Das Messer 78a am unteren Ende 76a der Schabkante 74a greift an dem Gewebe 84 an und hebt dieses entlang der Drehrichtung des Pfeils 86 von der Außenfläche 66 der Elektrode 22 ab. Das teilweise abgekratzte Gewebe 84 kann sich, wie in Fig. 4 gezeigt, über den Abstreifer 34 legen, bevor es nach seinem vollständigen Ablösen von der Außenfläche 66 der Elektrode 22 vom Abstreifer 34 abfällt und dann durch das Saugrohr 40 abgesaugt wird.

Die Elektrode 22 kann auch entlang eines Pfeils 88 um die Längsachse 80 des medizinischen Instruments 10 gedreht werden, so dass das Gewebe, das sich benachbart zur Schabkante 74b befindet, von der Außenfläche 66 der Elektrode 22 entfernt wird.

Das Drehen der Elektrode 22 um einen Drehwinkel von 180° um die Längsachse 80 des medizinischen Instruments 10 führt zu einem von der Schabkante 74a, b aus gerechnetem halbumfänglichen Reinigen der Außenfläche 66 der Elektrode 22, wodurch ein Entfernen der Verschmutzungen 84 von der gesamten Elektrodenaußenfläche 66 durch ein Drehen der Elektrode 22 um jeweils etwa 180° in beide Drehrichtungen 86, 88 erzielt wird. Es ist insbesondere durch das Vorhandensein der zwei Schabkanten 74a, b nicht notwendig, die Elektrode 22 um einen Winkel von 360° um die Längsachse 80 zu drehen, um die Verschmutzungen 84, die benachbart zu einer der Schabkanten 74a, b sind, durch die jeweils andere Schabkante 74a, b zu entfernen.

## Patentansprüche

1. Medizinisches Instrument zum Koagulieren von Gewebe, mit einem Elektrodenträger (42) und mit einer mit Hochfrequenzstrom beaufschlagbaren Elektrode (22) an einem distalen Ende (44) des Elektrodenträgers (42), wobei die Elektrode (22) rohrförmig ausgebildet ist und an einem distalen Ende (58) eine Öffnung (60) aufweist, und mit einem Abstreifer (34), der unmittelbar benachbart zur Elektrode (22) angeordnet ist, wobei der Abstreifer (34) und die Elektrode (22) zum Entfernen von Verschmutzungen (84) an der Elektrode (22) relativ zueinander bewegbar sind, **dadurch gekennzeichnet, dass** der Abstreifer (34) das distale Ende (58) der Elektrode (22) so umgreift, dass der Abstreifer (34) von der Außenseite der Elektrode (22) kommend in die Öffnung (60) der Elektrode (22) elngreift.

2. Instrument nach Anspruch 1, **dadurch gekennzeichnet, dass** der Abstreifer (34) und die Elektrode (22) quer zu einer Längsrichtung des Elektrodenträgers (42) relativ zueinander bewegbar sind.

3. Instrument nach Anspruch 1 oder 2, **dadurch gekennzeichnet, dass** sich der Abstreifer (34) in Längsrichtung zumindest entlang eines Teilbereichs der Elektrode (22) erstreckt.

4. Instrument nach einem der Ansprüche 1 bis 3, **dadurch gekennzeichnet, dass** sich der Abstreifer (34) nach distal hin verjüngt.

5. Instrument nach einem der Ansprüche 1 bis 4, **dadurch gekennzeichnet, dass** der Abstreifer (34) das distale Ende (58) der Elektrode (22) hakenförmig umgreift.

6. Instrument nach einem der Ansprüche 1 bis 5, **dadurch gekennzeichnet, dass** der Abstreifer (34) eine der Elektrode (22) zugewandte Schabkante (74a, b) aufweist, die als Messer (78a, b) ausgebildet ist.

7. Instrument nach einem der Ansprüche 1 bis 6, **dadurch gekennzeichnet, dass** der Abstreifer (34) zwei der Elektrode (22) zugewandte Schabkanten (74a, b) aufweist, die jeweils als Messer (78a, b) ausgebildet sind.

8. Instrument nach einem der Ansprüche 1 bis 7, **dadurch gekennzeichnet, dass** der Abstreifer (34) plattenförmig ist, wobei der Abstreifer (34) die Elektrode (22) teilumfänglich umgibt.

9. Instrument nach Anspruch 8, **dadurch gekennzeichnet, dass** der Abstreifer (34) die rohrförmige Elektrode (22) in Umfangsrichtung um die Längsachse der Elektrode (22) über einen Winkelbereich von weniger als etwa 60°, vorzugsweise über einen Winkelbereich von weniger als etwa 45° und weiter vorzugsweise über einen Winkelbereich von weniger als etwa 30° umgibt.

10. Instrument nach einem der Ansprüche 1 bis 9, **gekennzeichnet durch** ein Außenrohr (38), an dessen distalem Ende (46) der Abstreifer (34) angeordnet ist, wobei der Elektrodenträger (42) in dem Außenrohr (38) aufgenommen ist, und wobei der Elektrodenträger (42) und das Außenrohr (38) relativ zueinander drehbar sind.

11. Instrument nach Anspruch 10, **dadurch gekennzeichnet, dass** das Außenrohr (38) drehfest ist und der Elektrodenträger (42) relativ zum Außenrohr (38) drehbar ist.

12. Instrument nach einem der Ansprüche 1 bis 11, **dadurch gekennzeichnet, dass** der Elektrodenträger (42) als Saugrohr (40) ausgebildet ist.

## Claims

1. A medical instrument for coagulation of tissue, comprising an electrode carrier (42) and, at a distal end (44) of the electrode carrier (42), an electrode (22) that can be supplied with high-frequency current, wherein the electrode (22) is tubular and has an opening (60) at a distal end (58), and a stripper (34) arranged directly adjacent to the electrode (22), said stripper (34) and said electrode (22) being able to be moved relative to each other in order to remove contaminants (84) from the electrode (22), **characterized in that** the stripper (34) engages round the distal end (58) of the electrode (22) such that the stripper (34) engages, coming from the outer side of the electrode (22) into the opening (60) of the electrode (22).

2. The instrument of Claim 1, **characterized in that** the stripper (34) and the electrode (22) can be moved relative to each other transverse to a longitudinal direction of the electrode carrier (42).

3. The instrument of to Claim 1 or 2, **characterized in that** the stripper (34) extends in the longitudinal direction at least along a partial area of the electrode (22).

4. The instrument of any one of Claims 1 through 3, **characterized in that** the stripper (34) tapers in the distal direction.

5. The instrument of any one of Claims 1 through 4, **characterized in that** the stripper (34) engages round the distal end (58) of the electrode (22) in a hook-shaped fashion.

6. The instrument of any one of Claims 1 through 5, **characterized in that** the stripper (34) has a scraper edge (74a, 74b) facing towards the electrode (22) and designed as a blade (78a, 78b).

7. The instrument of any one of Claims 1 through 6, **characterized in that** the stripper (34) has two scraper edges (74a, 74b) facing towards the electrode (22) and each designed as a blade (78a, 78b).

8. The instrument of any one of Claims 1 through 7, **characterized in that** the stripper (34) is plate-shaped, the stripper (34) extending around part of the circumference of the electrode (22).

9. The instrument of Claim 8, **characterized in that** the stripper (34) extends around the tubular electrode (22) with an angle range of less than approximately 60°, preferably with an angle range of less than approximately 45°, and more preferably with an angle range of less than approximately 30°.

10. The instrument any one of Claims 1 through 9, **characterized by** an outer tube (38) at whose distal end (46) the stripper (34) is arranged, the electrode carrier (42) being received in the outer tube (38), and the electrode carrier (42) and the outer tube (38) being able to be rotated relative to each other.

11. The instrument of Claim 10, **characterized in that** the outer tube (38) is rotationally fixed and the electrode carrier (42) can be rotated relative to the outer tube (38).

12. The instrument of any one of Claims 1 through 11, **characterized in that** the electrode carrier (42) is designed as a suction tube (40).

## Revendications

1. Instrument médical destiné à coaguler des tissus, comportant un porte-électrodes (42) et une électrode (22) alimentée par un courant à haute fréquence à une extrémité distale (44) du porte-électrode (42), l'électrode (22) étant de forme tubulaire et présentant à une extrémité distale (58) une ouverture (60), et un racleur (34) qui se situe dans le voisinage immédiat de l'électrode (22), le racleur (34) et l'électrode (22) pouvant être mobiles l'un par rapport à l'autre pour éliminer les encrassements (84) de l'électrode (22), **caractérisé en ce que** le racleur (34) entoure l'extrémité distale (58) de l'électrode (22), **en ce que** le racleur (34) entoure l'électrode (22) du côté externe de l'électrode (22) à partir de l'ouverture (60).

2. Instrument selon la revendication 1, **caractérisé en ce que** le racleur (34) et l'électrode (22) sont mobiles l'un par rapport à l'autre, perpendiculairement par rapport à un sens longitudinal du porte-électrode (42).

3. Instrument selon la revendication 1 ou 2, **caractérisé en ce que** le racleur (34) s'étend dans le sens longitudinal, au moins le long d'une zone partielle de l'électrode (22).

4. Instrument selon l'une des revendications 1 à 3, **caractérisé en ce que** le racleur (34) s'affine vers le plan distal.

5. Instrument selon l'une des revendications 1 à 4, **caractérisé en ce que** le racleur (34) entoure l'extrémité distale (58) de l'électrode (22) en forme de crochet.

6. Instrument selon l'une des revendications 1 à 5, **caractérisé en ce que** le racleur (34) est constitué d'une arête coupante (74a, b) orientée vers l'électrode (22) qui est conçue comme un couteau (78a, b).

7. Instrument selon l'une des revendications 1 à 6, **caractérisé en ce que** le racleur (34) présentent deux arêtes coupantes (74a, b) orientées vers l'électrode (22) qui sont conçues respectivement comme des couteaux (78a, b).

8. Instrument selon l'une des revendications 1 à 7, **caractérisé en ce que** le racleur (34) est en forme de plaque, le racleur (34) entourant partiellement le pourtour de l'électrode (22).

9. Instrument selon la revendication 8, **caractérisé en ce que** le racleur (34) entoure l'électrode tubulaire (22) dans le sens longitudinal autour de l'axe longitudinal de l'électrode (22) sur un angle inférieur à environ 60°, de préférence sur un angle inférieur à environ 45° et de manière particulièrement préférée, sur un angle inférieur à environ 30°.

10. Instrument selon l'une des revendications 1 à 9, **caractérisé par** un tube externe (38) à l'extrémité distale (46) duquel le racleur (34) est disposé, le porte-électrode (42) étant reçu dans le tube externe (38) et le porte-électrode (42) et le tube externe (38) pouvant tourner l'un par rapport à l'autre.

11. Instrument selon la revendication 10, **caractérisé en ce que** le tube externe (38) est fixe et le porte-électrode (42) peut tourner par rapport au tube externe (38).

12. Instrument selon l'une des revendications 1 à 11, **caractérisé en ce que** le porte-électrode (42) est conçu comme un tube d'aspiration (40).
